# EUROPÄISCHE PATENTSCHRIFT

(11) **EP 3 723 591 B1**
(45) Veröffentlichungstag und Bekanntmachung des Hinweises auf die Patenterteilung: **14.06.2023**
(21) Anmeldenummer: 18847188.2
(22) Anmeldetag: 13.12.2018
(51) Int. Cl.: A61B 5/00, A61N 5/06, A61B 5/145, A61B 5/1459

(54) **VORRICHTUNG ZUR REGISTRIERUNG UND ZUR ZERSTÖRUNG EINZELNER TUMORZELLEN, TUMORZELLENCLUSTER UND MIKROMETASTASEN IM BLUTKREISLAUF**
DEVICE FOR REGISTERING AND FOR DESTROYING INDIVIDUAL TUMOUR CELLS, TUMOUR CELL CLUSTERS, AND MICROMETASTASES IN THE BLOODSTREAM
DISPOSITIF POUR DÉTECTER ET DÉTRUIRE DES CELLULES TUMORALES INDIVIDUELLES, DES AGRÉGATS DE CELLULES TUMORALES ET DES MICROMÉTASTASES DANS LA CIRCULATION SANGUINE.

(30) Priorität: 14.12.2017 DE 102017129971
(43) Veröffentlichungstag der Anmeldung: 21.10.2020
(73) Patentinhaber: Schikora, Detlef, 66-015 Zielona Gora (PL); Weber, Michael, 37697 Lauenförde (DE)
(72) Erfinder: Schikora, Detlef, 66-015 Zielona Gora (PL); Weber, Michael, 37697 Lauenförde (DE)
(74) Vertreter: Bremer, Ulrich
(86) Internationale Anmeldenummer: PCT/DE2018/101015
(87) Internationale Veröffentlichungsnummer: WO 2019/114883

(56) Entgegenhaltungen:
- US-A1- 2009 213 362
- US-A1- 2010 198 081
- US-A1- 2012 136 258
- US-A1- 2012 259 154
- US-A1- 2014 031 647
- US-A1- 2017 143 233

## Beschreibung

Die Erfindung betrifft eine Vorrichtung zur Registrierung und zur Zerstörung einzelner Tumorzellen, Tumorzellencluster und Mikrometastasen im Blutkreislauf, die mit einer photoempfindlichen Substanz angereichert sind.

Mit den heute in der onkologischen Routinediagnostik zur Verfügung stehenden biophysikalischen Methoden, wie Kernspinresonanz (MRT; fMRI), Positronenemissiontomographie (PET), Ultraschall (US), Computertomographie (CT) und Röntgen sind Tumoren direkt nachweisbar, wenn sie im Mittel eine Tumormasse von 1 g aufweisen. Dies entspricht ca. 1 Milliarde (10 ⁹) Tumorzellen. Erst ab dieser Größe lassen sich auch Gewebeproben direkt entnehmen (Biopsie) und zu gesicherter Diagnostik verwenden.

Eine Tumormasse von 1 g stellt mithin die derzeit gültige diagnostische Schwelle dar. Die an dieser diagnostischen Schwelle ermittelten Befunde gelten onkologisch als "Früherkennung" von Tumorwachstum bzw. Metastasierung.

Mittels immunologischer Methoden lassen sich Tumoren nachweisen, die eine Tumormasse von 1 mg aufweisen und entsprechend aus ca. 1 Million (10⁶) Tumorzellen bestehen. Diese Methoden sind indirekter Natur, da sie auf der Bestimmung von Markersubstanzen beruhen. Tumormarker sind Substanzen, die von malignen Tumorzellen direkt gebildet werden oder deren Synthese in Nicht-Tumorzellen durch Tumorzellen induziert wird und die auch bei gesunden Personen vorkommen.

Treten Tumormarker in erhöhter Konzentration im Blut oder in anderen Körperflüssigkeiten (humorale Tumormarker) bzw. in oder auf Zellen (zelluläre Tumormarker) auf, ermöglichen sie Rückschlüsse auf das Vorliegen, den Verlauf und die Prognose einer Tumorerkrankung. Diese Tumormarker sind generell unspezifisch. So indiziert eine hohe Konzentration des Tumormarkers CEA im Blutbild, dass ein Lungenkarzinom oder ein Leberkarzinom oder ein Dickdarmkarzinom oder ein Schilddrüsenkarzinom oder ein Mammakarzinom oder ein Magenkarzinom vorliegen könnte.

Die nach einer Operation praktisch unvermeidbar noch im Körper vorhanden Tumorzellen bzw. Mikrometastasen können später Rückfälle verursachen. Weder während einer Operation noch danach lassen sich mittels Ultraschall oder Computer-oder Magnetresonanztomographie nach gegenwärtigem Stand der Technik solche kleinen Zellnester (so genannte Mikrometastasen) nachweisen .Die Erfahrung besagt jedoch, dass in diesem Krankheitsstadium, also nach beendeter Chemotherapie, das Risiko für einen Tumorrückfall und Metastasenbildung für praktisch alle Krebspatienten von relevanter Größenordnung und keineswegs vernachlässigbar klein ist. Die Medizin, insbesondere die Onkologie, bietet gegenwärtig für diese Krankheitsphase lediglich das Prinzip "hoffnungsvolle Beobachtung" an. Therapeutische Optionen sind nicht vorhanden, da eine diagnostische Grundlage dafür fehlt.

Das Dokument US2009213362 offenbart eine Vorrichtung, die eine diagnostische Erkennung von Tumorzellen im Blutkreislauf von Menschen und Säugetieren als auch deren therapeutische in-situ-Eliminierung unter Verwendung derselben Stahlungsquelle ermöglicht. Das Dokument US2014031647 beschreibt eine Vorrichtung zur Erkennung von Tumorzellen im Blut unter Verwendung von zwei in Blutflussrichtung angeordneten Strahlungsquellen.

Es ist daher Aufgabe der vorliegenden Erfindung, eine Vorrichtung zur Verfügung zu stellen, die sowohl die sichere diagnostische Erkennung einzelner Tumorzellen im Blutkreislauf von Menschen und Säugetieren als auch deren therapeutische in-situ-Eliminierung ermöglicht.

Grundlage der vorliegenden Erfindung ist die in der Krebstherapie international erfolgreich eingesetzte, schulmedizinisch anerkannte photodynamische Diagnostik (PDD) sowie die photodynamische Therapie (PDT).

Die PDD und auch die PDT beruhen auf der optischen Anregung photoempfindlicher Substanzen, die dem Organismus topisch oder systemisch per Infusion zugeführt werden und die sich in Tumorzellen anreichern, da diese, unabhängig von der Tumorart, immer eine höhere Zellstoffwechselrate als normale, gesunde Zellen aufweisen.

Die optische Anregung derartiger lichtempfindlicher Substanzen (Photosensitizer) führt zur Bildung von reaktivem Sauerstoff (Singulet-Sauerstoff) in der Tumorzelle und zur oxidativen Zerstörung von essentiellen Zellbestandteilen (Mitochondrien, Zellwände) und endet mit einem nekrotischen oder apoptotischen Zelluntergang. Dieser Vorgang wird theoretisch durch das Jablonski-Diagramm beschrieben. (A. Jablonski: Efficiency of anti-Stokes fluorecence in dyes, Nature Bd 131, (1933), 839-840)

Das Jablonski-Diagramm besagt, dass der Prozess der Bildung reaktiver oxidativer Spezies durch die Entstehung und Emission von Photonen begleitet und charakterisiert ist. So entsteht bei der Relaxation des jeweiligen lichtempfindlichen Moleküls vom einem ersten angeregten Zustand (single state) in den Grundzustand Fluoreszenzstrahlung, beim Übergang vom einem weiteren angeregten Zustand (triplet state) in den Grundzustand Phosphoreszenzstrahlung und bei der Relaxation von angeregtem Singulet-Sauerstoff zu molekularem Sauerstoff charakteristische Lumineszenzstrahlung.

Diese drei verschiedenen Photonenemissionen stellen ein Charakteristikum für einen photodynamischen Prozess (PDD und PDT) dar und sind der eindeutige physikalische Nachweis eines aktivierten photodynamischen Vorgangs. Dieser Vorgang spielt sich in jeder Tumorzelle ab, die mit einem Photosensitizer angereichert und optisch aktiviert wurde, darüber hinaus natürlich auch in jeder Mikrometastase und in jedem makroskopischen Tumor, unabhängig von der Tumorart.

Die Intensität der emittierten Strahlung ist direkt proportional zu der Anzahl der photodynamisch aktivierten Tumorzellen.

Mittels moderner optischer Detektoren ist beim gegenwärtigen Stand der Technik der Nachweis einzelner Photonen möglich (A. Korneev, G. Goltsman, W. H. P. Pernice "Single Photon Counting: Photonic integration meets single photon detection" LaserFocus World V.51Is.5.05/05/2015 ).

Für den Nachweis von Einzelphotonen werden Detektoren mit hohem Gain eingesetzt. Dazu gehören Photomultiplier (PMT) und Avalanche Photodioden (APD).

Die oben gestellte Aufgabe wird erfindungsgemäß mit einer Vorrichtung gelöst, die die Merkmale des Anspruchs 1 aufweist.

Damit wird die diagnostische Schwelle der Tumoridentifikation und Tumortherapie drastisch abgesenkt, da die Diagnose und Therapie von einzelnen Tumorzellen oder Mikrometastasen möglich ist. Es kann eine Tumorfrüherkennung im wahrsten Wortsinne durchgeführt werden. Dies gilt generell für alle Patienten mit erhöhtem familiärem Krebsrisiko im Sinne einer prophylaktischen Therapie als auch für alle Tumorpatienten nach adjuvanter oder palliativer Chemo-/Strahlentherapie und für die Veterinärmedizin.

Die Erfindung wird nachstehend an Ausführungsbeispielen näher erläutert. In der dazu gehörigen Zeichnung zeigt in schematischer Weise:
- Fig. 1: eine erste Ausführungsform einer erfindungsgemäßen Vorrichtung mit extravasaler Anregung von Tumorzellen und intravasaler Detektion der emittierten Strahlung,
- Fig. 2: eine zweite Ausführungsform einer erfindungsgemäßen Vorrichtung mit intravasaler Anregung von Tumorzellen und extravasaler Detektion der emittierten Strahlung,
- Fig. 3: eine dritte Ausführungsform einer erfindungsgemäßen Vorrichtung, mit intravasaler Anregung von Tumorzellen und intravasaler Detektion der emittierten Strahlung, und
- Fig. 4: eine vierte Ausführungsform einer erfindungsgemäßen Vorrichtung, mit extravasaler Anregung von Tumorzellen und extravasaler Detektion der emittierten Strahlung.

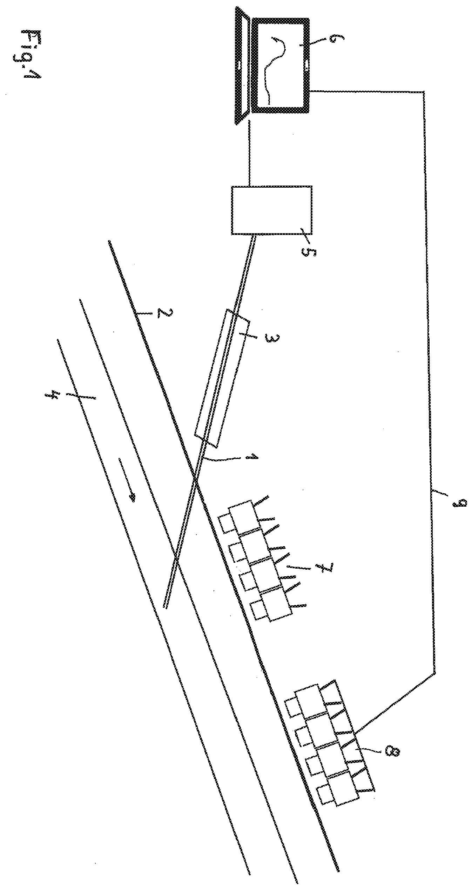

In der Ausführungsform gemäß Fig. 1 weist eine erfindungsgemäße Vorrichtung einen Lichtwellenleiter 1 auf, der mittels eines geeigneten auf der Haut 2 eines Patienten festgelegten peripheren Venenkatheters 3 (z. B. einer Braunüle) mit seinem Eingang in einer oberflächennahen Vene 4 positioniert ist. Der Ausgang des Lichtwellenleiters 1 ist mit einem hinsichtlich seiner spektralen Lage und Empfindlichkeit angepassten, hochempfindlichen Fluoreszenz-Spektrometer oder Ramann-Spektrometer 5 verbunden. Das Spektrometer 5 ist seinerseits an einen Computer 6 angeschlossen. Entlang der Vene 4 ist außen auf der Haut 2 ein beispielsweise 6 cm langes und 1,5 cm breites LD/LED-Feld 7 so angeordnet, dass die Eingangsfläche des Lichtwellenleiters 1 ca. 10 mm in das Strahlungsfeld des LD/LED-Feldes 7 hineinragt.

Gearbeitet wird mit der beschriebenen Vorrichtung wie folgt:
Als Photosensitizer kommt Indocyangrün (ICG) zum Einsatz. Durch einen peripheren Infusionskatheter wird eine dem Körpergewicht des Patienten angepasste Menge ICG in den Blutkreislauf infundiert. Sind in dem Blutkreislauf noch Tumorzellen vorhanden, werden diese den Photosensitizer im Vergleich zu den normalen Blutzellen stärker anreichern, da sie über eine höhere Stoffwechselrate verfügen.

Nach einer Einwirkzeit von ca. 3 Stunden wird der periphere Venenkatheter 3 gelegt, bspw. in die Vena basilica oder eine andere oberflächliche Arm- oder Handvene 4. In den Venenkatheter 3 wird der Lichtwellenleiter 1 eingeführt und wie oben beschrieben plaziert.

Es ist bekannt, dass ICG durch eine Strahlung mit einer Wellenlänge von 780 nm optisch angeregt wird. Eine Infrarotstrahlung mit eben dieser Wellenlänge emittiert das LD/LED-Feld 7. Wird dieses eingeschaltet, wird das im Blutstrom gleichmäßig verteilte ICG aktiviert. Die dabei entstehende Fluoreszenz-, Phosphoreszenz-, und Lumineszenzstrahlung tritt in die Strahlungseintrittsfläche des Lichtwellenleiters 1 ein und wird von dort zum Spektrometer 5 geleitet. Im Spektrometer 5 wird diese Strahlung analysiert und als funktionaler Zusammenhang der Strahlungsintensität über der Wellenlänge dargestellt. Das auf dies Weise generierte Basisspektrum wird mittels einer geeigneten Software von sich selbst subtrahiert, so dass ein Differenzspektrum erzeugt und in dem angeschlossenen Computer 6 angezeigt wird, dessen Intensität als Funktion der Wellenlänge gleich Null ist.

Strömt nun eine mit ICG angereicherte Tumorzelle oder ein Tumorzellencluster in den Strahlungsbereich des LD/LED-Feldes 7 ein, emittiert diese Fluoreszenz-, Phosphoreszenz- und Lumineszenzstrahlung, deren Intensitäten in jedem Fall größer sind als Null und demzufolge als Intensitätspeaks im Spektrum erscheinen.

Die Messung des Spektrums erfolgt kontinuierlich über einen Zeitraum t1, der so gewählt wird, dass das gesamte Blutvolumen mehrere Mal, vorzugsweise zwei- bis dreimal, den Lichtwellenleiter 1 und das Strahlungsfeld des LD/LED- Feldes 7 passiert hat.

Weist das für den Messzeitraum t1 registrierte Spektrum keine Peaks auf, ist das ein sicheres Zeichen, dass keine über der Basisintensität liegende Strahlung detektiert wurde und demzufolge keine im Blutstrom vagabundierenden Tumorzellen vorhanden sind.

Weist das für den Messzeitraum t1 registrierte Spektrum dagegen Peaks bei 830 nm (Fluoreszenzstrahlung) und/oder 945 nm (Phosphoreszenzstrahlung) und/oder 1245 nm (Lumineszenzstrahlung) auf, deutet dies auf das Vorhandensein von vagabundierenden Tumorzellen im Blutstrom hin. Die photodynamische Diagnostik (PDD) des Blutstromes ist damit beendet.

Sind in der PDD Strahlungspeaks nachgewiesen, wird zur Zerstörung dieser Tumorzellen die photodynamische Therapie (PDT) angewendet, indem ein weiteres, beispielsweise. 6 cm langes und 1,5 cm breites LD/LED-Feld 8 außen, entlang der Vene 4 in Blutflussrichtung hinter dem LD/LED-Feld 7 angeordnet wird, dessen Emission genau auf die Wellenlänge der in der PDD detektierten Fluoreszenzstrahlung, nämlich 830 nm, eingestellt ist. Die Aktivierung des LD/LED-Feld 8 für einen Behandlungszeitraum t1 erfolgt rechnergesteuert bei Registrierung eines Strahlungspeaks im Computer 6, wozu der Computer 6 und das LD/LED-Feld 8 über eine Leitung 9 miteinander verbunden sind. Die nur wenig in der Haut 2 absorbierte 830 nm Strahlung tritt mit nahezu voller Intensität in die Blutbahn ein und zerstört die dort vagabundierenden Tumorzellen.

Durch Streuvorgänge resultiert ein Strahlungsfeld des LD/LED-Feldes 8 von ca. 10 cm Länge. Bei einer mittleren venösen Fließgeschwindigkeit von ca. 10 cm/s ( z.B. vena femoralis superior ) resultiert eine Aufenthaltszeit einer Tumorzelle im Strahlungsfeld von 1 s. Bei einer angenommenen mittleren venösen Flussrate von 400 -900 ml/min. resultiert ein Behandlungszeitraum t1 = 7-15 min für einen Durchlauf, d.h. eine effektive Behandlungszeit von 15 -30 min.

Das Ergebnis der PDT kann danach unmittelbar überprüft werden, indem die oben beschriebene spektrale Blutanalyse (PDD) erneut durchgeführt wird.

Alternativ kann die Entfernung der diagnostizierten Tumorzellen natürlich auch in einem späteren Behandlungsschritt mit den Behandlungsmethoden erfolgen, die in der Onkologie eingeführt sind (Chemotherapie, Strahlentherapie).

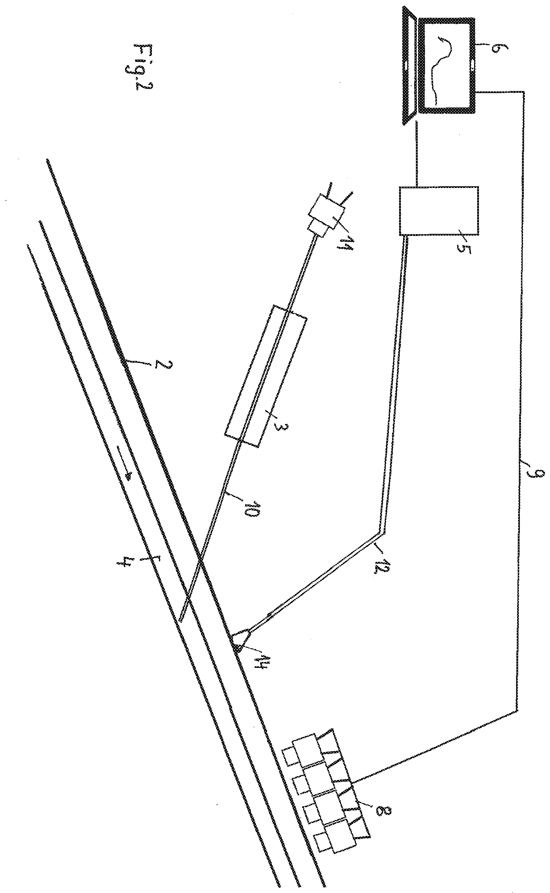

In der Ausführungsform gemäß Fig. 2 weist eine erfindungsgemäße Vorrichtung einen Lichtwellenleiter 10 auf, der mittels eines geeigneten auf der Haut 2 eines Patienten festgelegten peripheren Venenkatheters 3 (z. B. einer Braunüle) mit seinem Ausgang in einer oberflächennahen Vene 4 positioniert ist. In den Eingang des Lichtwellenleiters 10 ist eine LD oder LED 11 als Strahlungsquelle eingebunden. Über dem Ausgang des intravenösen Lichtwellenleiters 10 ist auf der Haut 2 des Patienten der Eingang eines weiteren Lichtwellenleiters 12 fixiert, der konisch gestaltet ist, um die Strahlungseintrittssläche zu vergrößern. Der Ausgang des Lichtwellenleiters 12 ist mit einem hinsichtlich seiner spektralen Lage und Empfindlichkeit erfindungsgemäß angepassten, hochempfindlichen Fluoreszenz-Spektrometer oder Ramann-Spektrometer 5 verbunden. Das Spektrometer 5 ist seinerseits an einen Computer 6 angeschlossen.

Gearbeitet wird mit der beschriebenen Vorrichtung wie folgt:
Als Photosensitizer kommt Chlorin e6 zum Einsatz. Durch einen peripheren Infusionskatheter wird eine dem Körpergewicht des Patienten angepasste Menge Chlorin e6 in den Blutkreislauf infundiert. Sind in dem Blutkreislauf noch Tumorzellen vorhanden, werden diese den Photosensitizer im Vergleich zu den normalen Blutzellen stärker anreichern, da sie über eine höhere Stoffwechselrate verfügen.

Nach einer Einwirkzeit von ca. 3 Stunden wird der periphere Venenkatheter 3 gelegt, bspw. in die Vena basilica oder eine andere oberflächliche Arm- oder Handvene 4. In den Venenkatheter 3 wird der Lichtwellenleiter 10 eingeführt und wie oben beschrieben plaziert.

Es ist bekannt, dass Chlorin e6 durch Licht mit einer Wellenlänge von 405 nm (purpurblau) optisch angeregt wird. Licht dieser Wellenlänge emittiert die in den Lichtwellenleiter 10 eingebundene LD/LED 11 bei einer Leistung von 50 mW. Wird diese eingeschaltet, wird das im Blutstrom gleichmäßig verteilte Chlorin e6 aktiviert. Die dabei entstehende Fluoreszenz-, Phosphoreszenz-,und Lumineszenzstrahlung tritt in die Eingangsfläche des Lichtwellenleiters 12 ein und wird von dort zum Spektrometer 5 geleitet. Im Spektrometer 5 wird diese Strahlung analysiert und als funktionaler Zusammenhang der Strahlungsintensität über der Wellenlänge dargestellt. Das auf dies Weise generierte Basisspektrum wird mittels einer geeigneten Software von sich selbst subtrahiert, so dass ein Differenzspektrum erzeugt und in einem angeschlossenen Computer 6 angezeigt wird, dessen Intensität als Funktion der Wellenlänge gleich Null ist.

Strömt nun eine mit Chlorin e6 angereicherte Tumorzelle oder ein Tumorzellencluster in das intravasale 405 nm Strahlungsfeld des Lichtwellenleiters 10 ein , emittiert diese Fluoreszenz-, Phosphoreszenz-und Lumineszenzstrahlung, deren Intensitäten in jedem Fall größer sind als Null und demzufolge als Intensitätspeaks im Spektrum erscheinen.

Die Messung des Spektrums erfolgt kontinuierlich über einen Zeitraum t1, der so gewählt wird, dass das gesamte Blutvolumen mehrere Male (zweibis dreimal) das intravasale 405nm Strahlungsfeld passiert hat.

Weist das für den Messzeitraum t1 registrierte Spektrum keine Peaks auf, ist das ein sicheres Zeichen, dass keine über der Basisintensität liegende Strahlung detektiert wurde und demzufolge keine im Blutstrom vagabundierenden Tumorzellen vorhanden sind.

Weist das für den Messzeitraum t1 registrierte Spektrum dagegen Peaks bei 660 nm (Fluoreszenzstrahlung) und/oder 840 nm (Phosphoreszenzstrahlung) und/oder 1245 nm (Lumineszenzstrahlung) auf, deutet dies auf das Vorhandensein von vagabundierenden Tumorzellen im Blutstrom hin. Die photodynamische Diagnostik (PDD) des Blutstromes ist damit beendet.

Sind in der PDD Strahlungspeaks nachgewiesen, wird zur Zerstörung dieser Tumorzellen die photodynamische Therapie (PDT) angewendet, indem ein beispielsweise. 6 cm langes und 1,5 cm breites LD/LED-Feld 8 au-ßen, entlang der Vene 4 in Blutflussrichtung hinter dem Strahlungsfeld des Lichtwellenleiters 10 angeordnet wird, dessen Emission genau auf die Wellenlänge der in der PDD detektierten Fluoreszenzstrahlung, nämlich 660 nm, eingestellt ist. Die Aktivierung des LD/LED-Feld 8 für einen Behandlungszeitraum t1 erfolgt rechnergesteuert bei Registrierung eines Strahlungspeaks im Computer 6, wozu der Computer 6 und das LD/LED-Feld 8 über eine Leitung 9 miteinander verbunden sind. Die nur wenig in der Haut 2 absorbierte 660 nm Strahlung tritt mit nahezu voller Intensität in die Blutbahn ein und zerstört die dort vagabundierenden Tumorzellen.

Durch Streuvorgänge resultiert ein Strahlungsfeld des LD/LED-Feldes 8 von ca. 10 cm Länge. Bei einer mittleren venösen Fließgeschwindigkeit von ca. 10 cm/s (z.B. vena femoralis superior) resultiert eine Aufenthaltszeit einer Tumorzelle im Strahlungsfeld von 1 s. Bei einer angenommenen mittleren venösen Flussrate von 400 -900 ml/min. resultiert ein Behandlungszeitraum t1 = 7-15 min für einen Durchlauf, d.h. eine effektive Behandlungszeit von 15 bis 30 min.

Das Ergebnis der PDT kann danach unmittelbar überprüft werden, indem die oben beschriebene spektrale Blutanalyse (PDD) erneut durchgeführt wird.

Alternativ kann die Entfernung der diagnostizierten Tumorzellen natürlich auch in einem späteren Behandlungsschritt mit den Behandlungsmethoden erfolgen, die in der Onkologie eingeführt sind (Chemotherapie, Strahlentherapie).

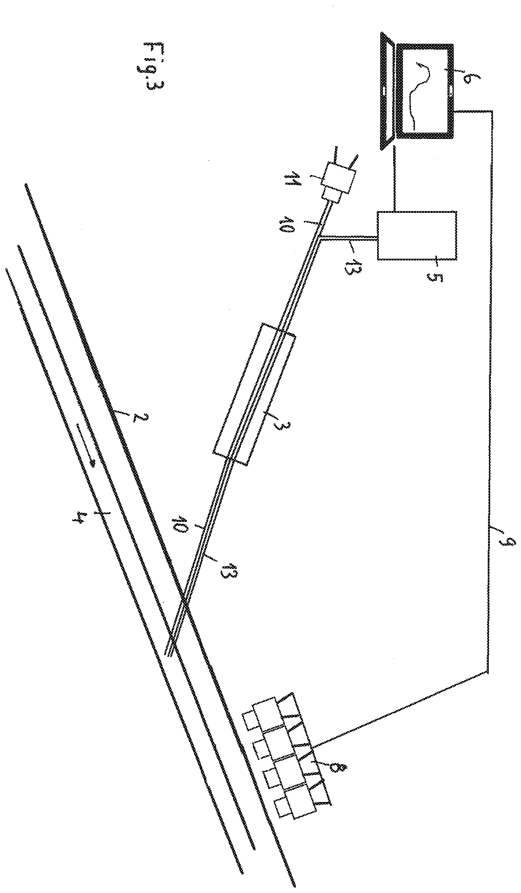

In der Ausführungsform gemäß Fig. 3 weist eine erfindungsgemäße Vorrichtung einen ersten Lichtwellenleiter 10 auf, der mittels eines geeigneten auf der Haut 2 eines Patienten festgelegten peripheren Venenkatheters 3 (z. B. einer Braunüle) mit seinem Ausgang in einer oberflächennahen Vene 4 positioniert ist. In den Eingang des ersten Lichtwellenleiters 10 ist eine LD oder LED 11 als Strahlungsquelle eingebunden. Mittels des auf der Haut 2 des Patienten festgelegten peripheren Venenkatheters 3 ist ein zweiter Lichtwellenleiter 13 parallel zum ersten Lichtwellenleiter 10 intravasal derart in der Vene 4 positioniert, dass sein Eingang im Lichtfeld des ersten Lichtwellenleiters 10 liegt und die durch optische Anregung entstehende Strahlung aufnimmt.

Der Ausgang des Lichtwellenleiters 13 ist mit einem hinsichtlich seiner spektralen Lage und Empfindlichkeit erfindungsgemäß angepassten, hochempfindlichen Fluoreszenz-Spektrometer oder Ramann-Spektrometer 5 verbunden. Das Spektrometer 5 ist seinerseits an einen Computer 6 angeschlossen.

Gearbeitet wird mit der beschriebenen Vorrichtung wie folgt:
Als Photosensitizer kommt 5-alpha Lävulinsäure (5-ALA) zum Einsatz. Durch einen peripheren Infusionskatheter wird eine dem Körpergewicht des Patienten angepasste Menge 5-ALA in den Blutkreislauf infundiert. Sind in dem Blutkreislauf noch Tumorzellen vorhanden, werden diese den Photosensitizer im Vergleich zu den normalen Blutzellen stärker anreichern, da sie über eine höhere Stoffwechselrate verfügen.

Nach einer Einwirkzeit von ca. 3 Stunden wird der periphere Venenkatheter 3 gelegt, bspw. in die Vena basilica oder andere, oberflächliche Arm- oder Handvenen 4. In den Venenkatheter 3 werden die beiden Lichtwellenleiter 10 und 13 eingeführt und wie oben beschrieben platziert.

Es ist bekannt, dass 5-ALA durch Licht mit einer Wellenlänge von 405 nm (purpurblau) optisch angeregt wird. Licht dieser Wellenlänge emittiert die in den Lichtwellenleiter 10 eingebunden LD/LED 11 bei einer Leistung von 50 mW. Wird diese eingeschaltet, wird das im Blutstrom gleichmäßig verteilte 5-ALA aktiviert. Die dabei entstehende Fluoreszenz-, Phosphoreszenz- und Lumineszenzstrahlung tritt in die Eingangsfläche des Lichtwellenleiters 13 ein und wird von dort zum Spektrometer 5 geleitet. Im Spektrometer 5 wird diese Strahlung analysiert und als funktionaler Zusammenhang der Strahlungsintensität über der Wellenlänge dargestellt. Das auf dies Weise generierte Basisspektrum wird mittels einer geeigneten Software von sich selbst subtrahiert, so dass ein Differenzspektrum erzeugt und in dem angeschlossenen Computer 6 angezeigt wird, dessen Intensität als Funktion der Wellenlänge = Null ist.

Strömt nun eine mit 5-ALA angereicherte Tumorzelle oder ein Tumorzellencluster in das intravasale 405 nm Strahlungsfeld ein , emittiert diese Fluoreszenz-, Phosphoreszenz- und Lumineszenzstrahlung, deren Intensitäten in jedem Fall größer sind als Null und demzufolge als Intensitätspeaks im Spektrum erscheinen.

Die Messung des Spektrums erfolgt kontinuierlich über einen Zeitraum t1, der so gewählt wird, dass das gesamte Blutvolumen mehrere Male (zwei bis dreimal) das intravasale 405 nm Strahlungsfeld passiert hat (siehe oben zwischen 15 min-30 min).

Weist das für den Messzeitraum t1 registrierte Spektrum keine Peaks auf, ist das ein sicheres Zeichen, dass keine über der Basisintensität liegende Strahlung detektiert wurde und demzufolge keine im Blutstrom vagabundierenden Tumorzellen vorhanden sind.

Weist das für den Messzeitraum t1 registrierte Spektrum dagegen Peaks bei 630 nm (Fluoreszenzstrahlung) und/oder 780 nm (Phosphoreszenzstrahlung) und/oder 1245 nm (Lumineszenzstrahlung) auf, deutet dies auf das Vorhandensein von vagabundierenden Tumorzellen im Blutstrom hin. Die photodynamische Diagnostik (PDD) des Blutstromes ist damit beendet.

Sind in der PDD Strahlungspeaks nachgewiesen, wird zur Zerstörung dieser Tumorzellen die photodynamische Therapie (PDT) angewandt, indem ein bspw. 6 cm langes und 1,5 cm breites extravasal außen, entlang der gro-ßen, oberflächlichen Vene 4 in Blutflussrichtung nach dem intravasalen 405 nm Strahlungsfeld des Lichtwellenleiters 10 angeordnetes LD/LED-Feld 8 mit einer Emissionswellenlänge von 630 nm und einer Leistungsdichte von 200 mW/cm2 für einen Behandlungszeitraum = t1 aktiviert wird. Die 630 nm Strahlung hoher Leistungsdichte tritt in die Blutbahn ein und zerstört die dort vagabundierenden Tumorzellen.

Das Ergebnis der PDT kann danach unmittelbar überprüft werden, indem die oben beschriebene spektrale Blutanalyse (PDD) erneut durchgeführt wird.

Alternativ kann die Entfernung der diagnostizierten Tumorzellen natürlich auch in einem späteren Behandlungsschritt mit den Behandlungsmethoden erfolgen, die in der Onkologie eingeführt sind ( Chemotherapie, Strahlentherapie ).

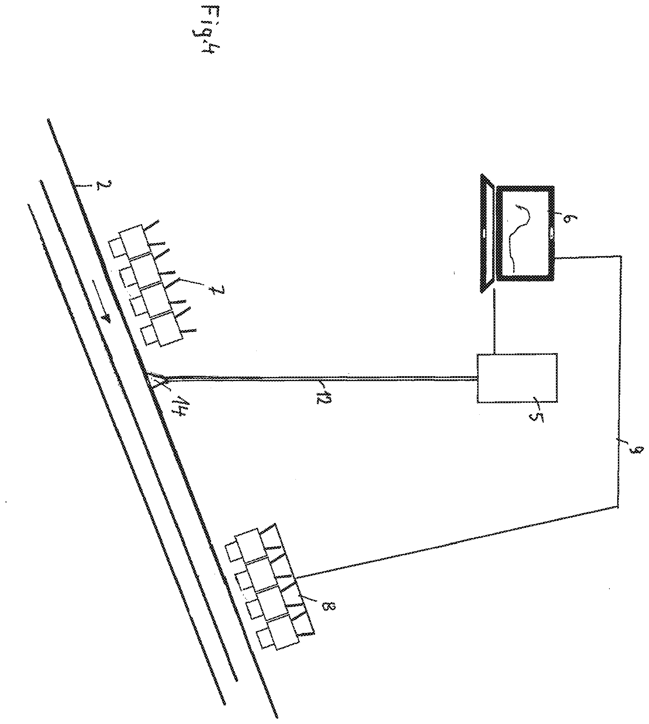

In der Ausführungsform gemäß Fig. 4 weist eine erfindungsgemäße Vorrichtung ein entlang einer Vene 4 außen auf der Haut 2 eines Patienten angeordnetes beispielsweise 6 cm langes und 1,5 cm breites LD/LED-Feld 7 auf. In Blutflussrichtung gesehen nach dem LD/LED-Feld 7 sitzt der Eingang eines Lichtwellenleiters 12 auf der Haut 2. Der Eingang ist konisch ausgeführt, um die Strahlungseintrittsfläche zu vergrößern. Der Ausgang des Lichtwellenleiters 12 ist mit einem hinsichtlich seiner spektralen Lage und Empfindlichkeit erfindungsgemäß angepassten, hochempfindlichen Fluoreszenz-Spektrometer oder Ramann-Spektrometer 5 verbunden. Das Spektrometer 5 ist seinerseits an einen Computer 6 angeschlossen. In Blutflussrichtung nach dem Lichtwellenleiter 12 ist ein weiteres LD/LED-Feld 8 angeordnet, welches über eine Leitung 9 mit dem Computer 5 verbunden ist. Die Anregung einer mit einem Photosensitizer angereicherten Tumorzelle erfolgt mittels des LD/LED-Feldes 7, welches eine entsprechende Strahlung emittiert. Die dabei entstehende Fluoreszenz-, Phosphoreszenz-, und Lumineszenzstrahlung tritt in die Eingangsfläche des Lichtwellenleiters 12 ein und wird von dort zum Spektrometer 5 geleitet.

Der diagnostische Nachweis einer Tumorzelle und ihre Eliminierung erfolgt in gleicher Weise wie oben beschrieben. Als Photosensitizer kann einer der oben genannten oder ein anderer geeigneter Photosensitizer zum Einsatz kommen. Das gilt auch für die zuvor beschriebenen Ausführungsbeispiele, die auf die genannten Photosensitizer nicht beschränkt sind.

## Patentansprüche

1. Vorrichtung zur Registrierung und zur Zerstörung von einzelnen Tumorzellen, Tumorzellenclustern und Mikrometastasen im Blutkreislauf, die mit einer photoempfindlichen Substanz angereichert sind, wobei die Vorrichtung aufweist
- eine Strahlungsquelle (7, 11), die zu intravasaler oder extravasaler Anregung der photoempfindlichen Substanz ausgelegt ist,
- einen Lichtwellenleiter (1, 12, 13) zur intravasaler oder extravasaler Leitung einer Fluoreszenz- und/oder Phosphorenz- und/oder Lumineszenzstrahlung der angeregten Tumorzellen und/oder Tumorzellencluster und/oder Mikrometastasen
- ein mit dem Lichtwellenleiter (1, 12, 13) verbundenes Fluoreszenzspektrometer (5) hoher Empfindlichkeit, das zur Registrierung der emittierten Strahlung ausgelegt ist,
- einen mit dem Spektrometer (5) verbundenen Computer (6), der dazu ausgelegt ist, die empfangenen Peaks der emittierten Strahlung als Funktion der Zeit aufzuzeichnen,
wobei die Vorrichtung
- eine weitere intravasal oder extravasal wirkende Strahlungsquelle (8) aufweist, die in Blutflussrichtung hinter dem Lichtwellenleiter (1, 12, 13) angeordnet und mit dem Computer (6) verbunden ist, der dazu ausgelegt ist, bei Registrierung eines Strahlungspeaks ein Einschaltsignal an die zweite Strahlungsquelle (8) auszugeben, wobei diese weitere Strahlungsquelle (8) zur Zerstörung von Tumorzellen und/oder Tumorzellencluster und/oder Mikrometastasen mittels photodynamischer Therapie durch Emission von Licht in der Wellenlänge des Strahlungspeaks ausgelegt ist.

2. Vorrichtung nach Anspruch 1, **dadurch gekennzeichnet, dass** der Lichtwellenleiter (1, 12, 13) einen konischen Eingang (14) zur Vergrößerung der Strahlungseintrittsfläche hat.

## Claims

1. Device for registering and destroying individual tumor cells, tumor cell clusters, and micro metastases in the circulatory system, which are enriched with a photo-sensitive substance, the device comprising
- a radiation source (7, 11) adapted to intravascularly or extra vascularly excite the photo-sensitive substance
- a fiber optic cable (1, 12, 13) for intravascularly or extra vascularly conducting a fluorescence and/or phosphorescence- and/or luminescence radiation of the excited tumor cells and/or tumor cell clusters and/or micro metastases
- a high sensitivity fluorescence spectrometer (5), connected to said fiber optic cable (1, 12, 13), for registering the radiation emitted
- a computer (6), connected to said spectrometer (5), for recording the received peaks of the radiation emitted as a function of time, the device comprising
- a further radiation source (8) acting intravascularly or extra vascularly, arranged, in the direction of blood flow, downstream from said detector (1, 12, 13) and connected to said computer (6), adapted to put out a switch-on signal to said second radiation source (8) upon registering a radiation peak, this further radiation source (8) being adapted to destroying tumor cells and/or tumor cell clusters and/or micro metastases by means of photo-dynamic therapy by emission of light in the wavelength of the radiation peak.

2. Device according to claim 1, **characterized in that** said fiber optic cable (1, 12, 13) is provided with a conical inlet (14) so as to enlarge the surface for the entry of radiation.

## Revendications

1. Dispositif d'enregistrement et de destruction de cellules tumorales individuelles, d'amas de cellules tumorales et de micrométastases dans la circulation sanguine qui sont enrichies en une substance photosensible, le dispositif présentant
- une source de rayonnement (7, 11) qui est conçue pour une stimulation intravasculaire ou extravasculaire de la substance photosensible,
- un guide d'ondes lumineuses (1, 12, 13) pour un guidage intravasculaire ou extravasculaire d'un rayonnement fluorescent et/ou phosphorescent et/ou luminescent des cellules tumorales et/ou des amas de cellules tumorales et/ou des micrométastases stimulés,
- un spectrofluoromètre (5) à sensibilité élevée relié au guide d'ondes lumineuses (1, 12, 13), ledit spectrofluoromètre étant conçu pour l'enregistrement du rayonnement émis,
- un ordinateur (6) relié au spectromètre (5), ledit ordinateur étant conçu pour enregistrer les pics reçus du rayonnement émis comme fonction du temps, ledit dispositif présentant une source de rayonnement supplémentaire à action intravasculaire ou extravasculaire (8) qui est disposée derrière le guide d'ondes lumineuses (1, 12, 13) dans le sens de la circulation sanguine et reliée à l'ordinateur (6), ledit ordinateur étant conçu pour émettre un signal de mise sous tension vers la deuxième source de rayonnement lorsqu'un pic de rayonnement est enregistré, ladite source de rayonnement supplémentaire (8) étant conçue pour la destruction de cellules tumorales et/ou d'amas de cellules tumorales et/ou de micrométastases au moyen d'une thérapie photodynamique par émission de lumière dans la longueur d'onde du pic de rayonnement.

2. Dispositif suivant la revendication 1, **caractérisé en ce que** le guide d'ondes lumineuses (1, 12, 13) présente une entrée conique (14) pour agrandir la surface d'entrée du rayonnement.
